Europäisches Patentamt

(19) European Patent Office

Office européen des brevets

(11) Publication number: **0 095 889**
**A2**

# (12) EUROPEAN PATENT APPLICATION

(21) Application number: **83303013.3**

(51) Int. Cl.³: **A 61 K 7/035**

(22) Date of filing: **25.05.83**

(30) Priority: **28.05.82 US 383204**

(71) Applicant: **MAX FACTOR & CO, 1655 North McCadden Place, Holywood California 90028 (US)**

(43) Date of publication of application: **07.12.83 Bulletin 83/49**

(72) Inventor: **Lanzet, Monroe, 4688 Alonzo Avenue, Encino California 91316 (US)**
Inventor: **Lambrechts, John, 1825 North Vista Street, Hollywood California 90046 (US)**

(84) Designated Contracting States: **AT BE CH DE FR GB IT LI LU NL SE**

(74) Representative: **Harrison, David Christopher et al, MEWBURN ELLIS & CO 2/3 Cursitor Street, London EC4A 1BQ (GB)**

(54) Solid cosmetic compositions and methods of preparing them.

(57) A solid cosmetic composition is provided having improved pay-off qualities and resistance to breakage and cracking which is formed by mixing two liquid dispersion slurries, the first slurry being non-aqueous or substantially so and comprising calcium sulfate hemihydrate, a hydroxylic liquid and cosmetic oils, fragrances, colorants or fillers, and the second slurry comprising water insoluble inorganic or organic particulate materials, colorants and water; the slurries being combined in proportions such that the mixture contains water in excess of that sufficient to totally convert the calcium sulfate hemihydrate to calcium sulfate dihydrate. The mixture solidifies within about 30 minutes to form a smooth, solid cosmetic composition, but the separate slurries have long storage life and can readily be handled.

## SOLID COSMETIC COMPOSITIONS AND
## METHODS OF PREPARING THEM

The present invention is directed to solid cosmetic compositions which provide improved product release while retaining excellent strength and resistance to breakage.

There is a need in the art for improved solid cosmetic compositions which exhibit excellent release of colourants and filler materials (known as "pay-off properties" in the art) and fragrance, yet which exhibit sufficient physical integrity and strength after molding to be usable without breaking or cracking and without partial deterioration into dust when rubbed. There is also a need to solve the problem in the current art that the shade of certain colourants used, such as carmine lakes, often looks different in the cake as compared with the colour when rubbed onto the skin. Methods presently used to form moldable cosmetic compositions call for the use of essentially dry powder compositions which are then mixed with oily or waxy material or binding and are then compressed, usually with a hydraulic or mechanical press, to form solid cosmetics and toiletries.

Another method known in the prior art is to form a wet slurry (i.e. a liquid dispersion) which is either poured into a mould, extruded to form a stick or formed into a shape from a paste, then dried in an oven. These are then used as is, after removal from the mould, or subsequently sanded to the desired shape.

Some disadvantages of these methods are the requirements for hydraulic or mechanical presses, extrusion equipment and oven drying equipment. Furthermore, many compositions known in the prior art suffer from the disadvantages of being scratchy or dusty when rubbed against the skin, having poor or little pay-off properties, poor skin adhesion or easily breaking or cracking if dropped.

Methods of preparation of the solid cosmetic compositions of the prior art may also be inconvenient and difficult since the dry materials and the wet or waxy materials must be kept separate until they are placed in the container which will accommodate the finished product. This requires the movement and grinding of bulk solid materials within the processing plant which is more difficult to handle than liquids or pumpable dispersions.

Another disadvantage of known cosmetic compositions using calcium sulfate hemihydrate is that once water is added to a conventional dry blend consisting of the colour, fillers and calcium sulfate it must be used completely within an hour or less (depending upon the quantity of accelerators or retarders used) because the hydration reaction will occur and the cosmetic will either partially or totally solidify in the mixing or filling equipment. Moreover, the strength of the final cosmetic block may be lessened if pouring is not completed within fifteen minutes after addition of the water.

The present invention provides solid moldable cosmetic compositions and methods of preparations thereof which do not suffer from the above disadvantages, and which permit continuous addition of water to a calcium sulfate hemihydrate phase as needed to maintain filling, setting time and consistent strength of the ultimate solid product.

The method also prepares solid moldable cosmetic compositions utilizing pumpable slurries which because they are each non-solidifying may be prepared and stored until needed. It also allows multi-coloured cosmetic blocks to be prepared.

The present cosmetic compositions may include platelet minerals and organic polymer resins together with the calcium sulfate hemihydrate which provide good product release, skin adhesion and excellent strength in the presence of pigments.

The present invention is directed to moldable solid cosmetic compositions containing calcium sulfate dihydrate (gypsum) and organic or inorganic particulate material, such as platelet minerals, which produce improved pay-off properties and resistance to breakage and to

-4-

0095889

the method of preparing said compositions utilizing at least two pumpable slurries, the first of which (Slurry A) contains calcium sulfate hemihydrate and optionally cosmetic fragrances and/or a low HLB surfactant in a water miscible hydroxylic liquid selected from alkanols containing from 1 to 4 carbon atoms, a glycol containing from 2 to 8 carbon atoms or mixtures thereof; and a second pumpable slurry (Slurry B) which contains water, water insoluble particulate materials and, optionally, pigments and water insoluble organic polymer resins such as ureaformaldehyde polymer powders. The two slurries may be separately prepared and stored indefinitely. Slurry A may optionally contain small amounts of water, emollients, fragrance oils, microencapsulated oils, calcium sulfate dihydrate and other ingredients which either accelerate or retard the subsequent hydration reaction. Water is one of the latter; but Slurry A will not contain more than a very little water, say 5 percent maximum.

Preferred compositions may be made from these slurries having, when admixed, from 2 to 5 parts of hydroxylic liquid per 10 to 12 parts of calcium sulfate hemihydrate, 40 to 45 parts of water and 15 to 40 parts of particulate filler (encapsulated cosmetic ingredients may be included in this figure).  The remainder, to 100 parts (all parts being by weight) will be the cosmetic ingredient(s).   More preferred compositions are made from 3 to 4 parts of hydroxylic liquid, 11 parts of calcium sulfate hemihydrate, 41 to 43 parts of water and 19 to 38 parts of filler.

In preparing the compositions according to the present invention, Slurry A is mixed with Slurry B in proportions such that there is an amount of water present in excess of that sufficient to totally convert the calcium sulfate hemihydrate to calcium sulfate dihydrate, whereby the resultant pourable mixture solidifies within about thirty minutes to form a smooth, solid cosmetic composition.

While calcium sulfate has been previously used as a powder base in solid cake cosmetics, these had poor pay-off characteristics and were rough and scratchy to use. Also, in preparing such cosmetics the calcium sulfate and other solid components of the cosmetic have heretofore been kept separate from any liquid or waxy component until mixed for filling the final container.

See, for example, U. S. Patent 1,968,475 to Beckwith et al. A problem utilizing calcium sulfate is that when it is mixed with excess water it almost immediately sets up as Plaster of Paris (gypsum) and gives a rough, scratchy and grainy appearance and feel. If insufficient calcium sulfate is present, then the cosmetic block will crumble or break if dropped. If calcium sulfate hemihydrate is used in quantities sufficient to produce a strong cosmetic block, it will result in a cosmetic with insufficient pay-off properties.

The present invention obviates these problems by making use of the structural integrity provided by sufficient calcium sulfate hemihydrate in a pumpable slurry which may be stored indefinitely prior to utilization to form the final product.

According to the present invention, separate pumpable slurries are created, one of which comprises calcium sulfate hemihydrate dispersed in a substantially water-free liquid (Slurry A). Preferably such liquid may be hydroxylic liquid substantially miscible with water, selected from an alkanol containing from 1 to 4 carbon atoms, a glycol containing from 2 to 6 carbon atoms or mixtures thereof, for example, methanol, ethanol, isopropanol, or butylene glycol. This slurry may also contain up to about 20% by weight of various cosmetic materials such as fragrance oils, emollients, microencapsulated oils, surface active agent, emulsifiers, colorants and/or fillers. Preferred fillers are inorganic platelet materials such as micronized mica, bismuth oxychloride coated mica or titanium dioxide coated mica. Platey talc may also be used in forming stick compositions. Preferably the slurry may contain approximately 10 parts by weight of the hydroxylic liquid, 70 parts by weight of a mixture of cosmetic fragrances, colorants, or fillers. Suitable slurry thickening agents such as water and/or sorbitan sesquioleate may also be added. While it is necessary that the combination remain substantially water-free during storage, the presence of less than 5%, preferably less than 3% and most preferably about 0.5 to 2% by weight, of water is beneficial to control setting time of the final composition.

In a second pumpable slurry (Slurry B), there will be dispersed in water, various cosmetic colorants, microencapsulated fragrance or cosmetic oils and/or fillers, particulate materials such as the platey inorganic materials mica or talc, and/or surface active agents, gel retarding or accelerating chemicals. Preferred microencapsulated fragrance or cosmetic oil may be encapsulated in a urea formaldehyde resin shell (Aminoplast)®. Slurry B provides the water required for the hydration reaction which will take place when the pumpable slurries are combined to form the final product. The composition of this aqueous slurry will be selected to prevent hard packing during storage so that a constant composition may be achieved in slurry form upon simple agitation. Preferably the slurry may contain approximately equal parts by weight of water and solid materials comprising encapsulated material, colors and platey inorganic materials. A preferred composition is 55% by weight water, and about 45% by weight water insoluble platey organic or inorganic particulate material minerals such as mica or talc. The encapsulated material may be fragrance or emollient oil microencapsulated in urea formaldehyde resin.

The cosmetic fragrances and oils in either of the above described pumpable slurries may be microencapsulated. Each slurry may contain different colorants whereby mixture of the slurries a final uniformly colored or multicolored solid cosmetic composition may be obtained. The above described pumpable slurries may be separately stored for an indefinite period and may be transported through the production areas through pipes and pumps. When it is desired to form the final cosmetic compositions, all that is required is to combine the slurry containing the calcium sulfate hemihydrate (hereinafter called Slurry A) and the aqueous slurry (hereinafter called Slurry B) in proportions in excess of that sufficient to totally convert the calcium sulfate hemihydrate to calcium sulfate dihydrate. When the pumpable slurries are formulated in the preferred portions as described above, then it is preferred that 15 parts by weight of Slurry A be mixed with 85 parts by weight of Slurry B with sufficient agitation to assure uniformity. The mixture will solidify after combination in less than 30 minutes, usually in about 10 to 20 minutes, to form an improved

0095889

cosmetic composition.

The two pumpable slurry parts may be continuously combined by a proportioning pump that will maintain a constant combined slurry composition.

Because the setting time of the combined slurries is not instantaneous, the combined slurry may be handled and processed from about 10 to 30 minutes without solidification of product. This is particularly convenient for the cleaning of equipment such as pumps and lines with water prior to terminating production operations.

As set forth above, the presence of a small quantity of water in Part A is not detrimental and has been found to actually be advantageous in terms of controlling the setting time and in maintaining a more easily redispersible form after storage. Less than 3%, preferably about 0.5 to 2% water in Slurry A by weight is preferred.

Optionally an equal part of a low HLB surfactant may also be included in Slurry A to further improve redispersability. Preferably less than about 3% by weight of a surfactant having an HLB of 1.8 to 6.0, such as sorbitan sesquioleate, may be used.

Exemplary compositions according to the present inventions are set forth below.

Example 1

In order to test the effect of using various platelet minerals three compositions 1A, 1B and 1C shown below were formulated. The dry ingredients of each were preblended and enough water was added to each to make a pourable slurry. No hydroxylic liquid was used. Each slurry was poured into a separate mold while still fluid, and excess water was allowed to evaporate. It was found that platey talc, micronized mica and titanium dioxide coated mica all produced a gypsum composition having improved pay-off characteristics.

|  | #1A Parts By Wt. | #1B Parts By Wt. | #1C Parts By Wt. |
|---|---|---|---|
| Platey Talc | 30.0 | -0- | -0- |
| D&C color dry mix | 10.0 | 10.0 | 10.0 |
| Micronized mica | -0- | 30.0 | -0- |
| Titanium coated mica | -0- | -0- | 30.0 |
| $CaSO_4 \cdot 1/2H_2O$ | 10.0 | 10.0 | 10.0 |
| Water | Sufficient water to make a pourable slurry. | | |

-11-

Example 2

Slurries A and B were formulated as shown below:

|  | Parts by Wt. | (% by Wt. |
|---|---|---|
| Slurry A Color Gellant Phase |  | of Composition) |
|  |  |  |
| 1, 3 Butylene glycol | 20.0 | (40%) |
| Hydrocal® calcium sulfate hemihydrate | 20.0 | (40%) |
| D&C red #7 lake | 10.0 | (20%) |
|  |  | 100% |
|  |  |  |
| Slurry B Aqueous Phase |  |  |
|  |  |  |
| Deionized water | 50.0 | (55%) |
| Mica | 20.0 | (22.5%) |
| Platey talc | 20.0 | (22.5%) |
|  |  | 100.0% |

Combination Ratio

| Slurry A | 10 parts by weight |
|---|---|
| Slurry B | 90 parts by weight |

The pumpable slurries, Slurries A and B, were combined in the portions shown above with sufficient agitation to assure uniformity. The mixture solidified in 10 to 20 minutes after combination. The resulting cosmetic composition exhibited superior pay-off properties and resistance to breakage and cracking.

There was some compaction on standing of Slurry A. Also thickness of Slurry B caused it to be not easily mixed with A.

Example 3

The pumpable slurries, A and B, were formulated as shown
below:

| Slurry A Color Gellant Phase | % by Weight of Composition |
|---|---|
| Isopropanol | 21% |
| Water, deionized | 2 |
| Hydrocal® calcium sulfate hemihydrate | 75 |
| Sorbitan Sesquioleate. | 2 |
| | 100% |

| Slurry B Aqueous Phase | |
|---|---|
| Deionized water | 50% |
| Mica, micronized | 45 |
| D&C red #7 lake | 5 |
| | 100% |

Combination Ratio

| Slurry A | 15 parts by weight |
|---|---|
| Slurry B | 85 parts by weight |

Slurry A in Example 3, above corrected the compaction
observed in Example 2. Slurry A did not hard pack on
standing and with gentle agitation was restored to a
uniform pumpable slurry. Slurry B above was thinner
than Slurry B in Example 2, and easily pumpable. When
blended in the ratio shown above, A and B were quickly
combined with moderate agitation to assure uniformity.

0095889

The resultant mixture solidified in 20 to 30 minutes
after combination.

The resultant blend mixture comprises a blend of about
3.15 parts by weight of isopropanol, about 11.25 parts
calcium sulfate hemihydrate, about 0.3 parts sorbitan
sesquioleate, about 42.8 parts water, about 38.25 parts
mica, and a colorant.

Slurries A and B were formulated as shown below to
study the incorporation of eye shadow colors and
microencapsulated oil in the formulation of a solid
makeup cosmetic.

Example 4

|  |  |
|---|---|
| Slurry A - Gellant Phase | % by Weight of Composition |
| Isopropanol | 21.0% |
| Deionized water | 2.0 |
| Surfactant | 2.0 |
| Calcium sulfate hemihydrate | 75.0 |
|  | 100.0% |

|  |  |
|---|---|
| Slurry B - Color Phase |  |
| Deionized water | 48.0% |
| Isopropanol | 2.0 |
| Microencapsulated mineral oil (in urea formaldehyde resin shell 30µ) | 10.0 |
| Calcium sulfate dihydrate | 1.0 |
| Bismuth Oxychloride on mica | 22.0 |
| Carmine lake | 10.0 |
| Ferric blue | 4.0 |
| Black iron oxide | 2.0 |
| Titanium Dioxide | 1.0 |
|  | 100.0% |

Combination Ratio

|  |  |
|---|---|
| Slurry A | 15 parts by weight |
| Slurry B | 85 parts by weight |

·The resultant mixture above solidified in 20 to 30 minutes to form an eye shadow block with improved strength and excellent color pay-off. Moreover, when the make-up was buffed after application, the microencapsulated oil was released providing excellent water resistance, improved wear and a creamy application.

The resultant blended mixture of Example 4 comprises a blend of about 3.85 parts by weight isopropanol, about 11.25 parts calcium sulfate hemihydrate, about 0.3 parts surfactant, about 41 parts water, about 8.5 parts microencapsulated mineral oil, about 0.85 parts calcium sulfate dihydrate, about 18.7 parts bismuth oxychloride on mica, about 8.5 parts Carmine lake, about 3.4 parts Ferric blue, about 1.7 parts Black iron oxide, and about 0.85 parts titanium dioxide.

In order to study the composition as a fragrance sachet, slurries A and B as shown below were formulated to produce a solid block with good fragrance release but no rub-out (pay-off) of powder on skin.

## Example 5 (no pay-off)

| Slurry A - Gellant Phase | % by Weight of Composition |
|---|---|
| Ethyl alcohol | 4.0% |
| Perfume oil | 33.0 |
| Deionized water | 1.5 |
| Low HLB surfactant | 1.5 |
| Calcium sulfate hemihydrate | 60.0 |
| | 100.0% |

## Slurry B

| | |
|---|---|
| Deionized water | 40.0% |
| Platelet talc | 57.0 |
| Urea formaldehyde resin (30 μ shell) | 2.0 |
| Calcium sulfate dihydrate | 1.0 |
| | 100.0% |

## Combination Ratio

| | |
|---|---|
| Slurry A | 30 parts by weight |
| Slurry B | 70 parts by weight |

The composition of Example 5 comprises A and B in the 30:70 ratio shown solidified to produce a solid fragrance sachet containing both regular and microencapsulated perfume oil. Although there was little or no pay-off of product when rubbed on the skin, the composition released fragrance continuously for 2 weeks or more with excellent odor fidelity. The solid block did not interfere with either the release or the fidelity of the perfume oil used. Moreover, when the block was warmed, the intensity of fragrance release was enhanced without distorting the fragrance as it would if warmed by burning in conventional fragrance candle form.

In order to study the formulation of a solid dusting powder, slurries A and B shown below were developed to provide for both good pay-off on the skin and fragrance release.

Example 6 (with pay-off)

|  | % by Weight |
|---|---|
| Slurry A - Gellant Phase | of Composition |
| Ethyl alcohol | 20.0% |
| Perfume oil | 10.0 |
| Deionized water | 2.0 |
| Surfactant | 2.0 |
| Calcium sulfate hemihydrate | 66.0 |
|  | 100.0% |

| Slurry B | |
|---|---|
| Deionized water | 50.0% |
| Microencapsulated perfume oil in Aminoplast® resin (Shell-30µ) | 49.0 |
| Calcium sulfate dihydrate | 1.0 |
|  | 100.0% |

| Combination Ratio | |
|---|---|
| Slurry A | 30 parts by weight |
| Slurry B | 70 parts by weight |

After combination the mixture of slurry A and B solidified in about 15 minutes. When completely dry the resulting solid perfumed powder was easily rubbed on skin with a cosmetic puff. The skin deposition had good adhesion and produced a good fidelity fragrance effect on the skin and from the cake. Moreover, when the product on the skin was buffed, the microencapsulated fragrance cells ruptured to release an even greater fragrance intensity with continued fidelity compared to the original perfume oil.

The invention has been described with reference to specific embodiments thereof which produced the desired results. Other materials may be substituted for materials disclosed and amounts may be altered at the choosing of the formulator in order to achieve various additional attributes which would be within the skill and knowledge of those of ordinary skill in the art using the teachings of the above specification.

CLAIMS:

1. A method for preparing a solid cosmetic composition comprising mixing a first liquid dispersion which is free or substantially free of water and which includes calcium sulfate hemihydrate and a water miscible hydroxylic liquid which is an alkanol consisting of from 1 to 4 carbon atoms, a glycol containing from 2 to 8 carbon atoms or a mixture of two or more thereof;

with a second liquid dispersion comprising water insoluble particulate material and water in a proportion such that there is water present in an amount in excess of that sufficient to totally convert said calcium sulfate hemihydrate to calcium sulfate dihydrate, the mixture also containing a cosmetic ingredient, and allowing the mixture to solidify.

2. A method according to Claim 1, wherein the cosmetic ingredient was in the first liquid dispersion and is a low HLB surface active agent, emollient or fragrance oil, or microencapsulated oil.

3. A method according to Claim 1 or Claim 2, wherein the cosmetic ingredient was in the second liquid dispersion and is a water insoluble microencapsulated oil or colorant.

4.    A method according to any one of the preceding Claims, wherein said first liquid dispersion contains less than 5 percent by weight of water.

5.    A method according to any one of the preceding Claims, wherein said first liquid dispersion contains less than 3 percent of a surfactant having an HLB of 1.8 to 6.0.

6.    A method according to any one of the preceding Claims, wherein said water insoluble inorganic particulate material is a platelet material which is talc, mica, bismuth oxychloride coated mica or titanium dioxide coated mica.

7.    A method according to any one of the preceding Claims, wherein the hydroxylic liquid is butylene glycol, isopropanol, ethanol, methanol or a mixture of two or more thereof.

8.    A method according to any one of the preceding Claims, wherein said first liquid dispersion and said second pumpable liquid dispersion are mixed in proportions of 15 parts by weight to 85 parts by weight respectively.

9.    A cosmetic composition formed by combining a hydroxylic liquid which is an alkanol having 1 to 4 carbon atoms or a glycol containg 2 to 8 carbon atoms or a mixture thereof, calcium sulfate hemihydrate, insoluble particulate material, water sufficient to hydrate the calcium sulfate, and at least one cosmetic ingredient.

10. A cosmetic composition according to Claim 9 which is formed by combining 3 parts by weight of a hydroxylic liquid selected from an alkanol containing from 1 to 4 carbon atoms, a glycol containing from 2 to 8 carbon atoms or mixtures of such liquids,

about 11 parts by weight of calcium sulfate hemihydrate;

about 43 parts by weight water;

about 38 parts by weight water insoluble inorganic particulate material or about 38 parts of a mixture of water insoluble inorganic particulate material and encapsulated cosmetic fragrance of encapsulated emollient oil;

with the remainder being a cosmetic fragrance, colorant, surface active agent, microencapsulated oil, filler or a mixture of two or more thereof.

11. A composition according to Claim 10, wherein said encapsulated cosmetic fragrances or emollients are microencapsulated in ureaformaldehyde polymer resin.

12.     A cosmetic composition according to Claim 9 formed by combining about 4 parts by weight of a hydroxylic liquid which is an alkanol containing from 1 to 4 carbon atoms, a glycol containing from 2 to 8 carbon atoms or mixtures of two or more thereof;

about 11 parts by weight of calcium sulfate hemihydrate.

about 41 parts by weight of water;

about 19 parts by weight of water insoluble inorganic particulate material;

with the remainder a cosmetic fragrance, colorant, microencapsulated oil, surface active agent, filler, or mixture of two or more thereof.

13.     A liquid dispersion comprising about 40 percent by weight calcium sulfate hemihydrate, about 40 percent by weight of a hydroxylic liquid selected from an alkanol containing from 1 to 4 carbon atoms, a glycol containing from 2 to 8 carbon atoms or mixtures of such liquids; up to 5 percent by weight of water; and the remainder comprising cosmetic fragrances, emollients, colorants, microencapsulated oils or fillers.